# EUROPEAN PATENT SPECIFICATION

(11) **EP 2 581 030 B1**
(45) Date of publication and mention of the grant of the patent: **29.04.2015**
(21) Application number: 12770836.0
(22) Date of filing: 01.03.2012
(51) Int. Cl.: A61B 1/06

(54) **ENDOSCOPE APPARATUS**
ENDOSKOPGERÄT
APPAREIL D'ENDOSCOPE

(30) Priority: 11.04.2011 JP 2011087769
(43) Date of publication of application: 17.04.2013
(73) Proprietor: OLYMPUS MEDICAL SYSTEMS CORP., Tokyo 151-0072 (JP)
(72) Inventor: TAKEI, Shunji, Tokyo 151-0072 (JP); IGARASHI, Makoto, Tokyo 151-0072 (JP)
(74) Representative: Schicker, Silvia
(86) International application number: PCT/JP2012/055258
(87) International publication number: WO 2012/140970

(56) References cited:
- EP-A1- 2 301 418
- WO-A1-2010/044432
- JP-A- 2002 095 635
- JP-A- 2005 198 794
- JP-A- 2007 075 569
- US-A1- 2009 021 578
- US-A1- 2009 118 578
- TAKUJI YAMASAKI ET AL.: 'Kakudai Naishikyo wa Dokomade Hitsuyo ka? 3 I no Naishikyo (3) Chiryo Naishikyo no Tachiba kara' CLINICAL GASTROENTEROLOGY vol. 25, no. 8, 20 June 2010, pages 1129 - 1138

## Description

### Technical Field

The present invention relates to endoscope apparatuses, and more particularly, to an endoscope apparatus suitable for submucosal dissection.

### Background Art

Conventionally, various minimally invasive inspections and operations using an endoscope are performed in a medical field. Operators can insert an endoscope into a body cavity, observe an object, images of which are picked up by an image pickup apparatus provided at a distal end portion of an endoscope insertion portion and perform treatment on a lesioned region as required using a treatment instrument inserted in a treatment instrument channel. Surgery using an endoscope does not require abdominal operation or the like, thus having an advantage of reducing a physical burden on a patient.

An endoscope apparatus is configured by including an endoscope, an image processing apparatus connected to the endoscope and an observation monitor. An image pickup device provided at the distal end portion of the endoscope insertion portion picks up an image of the lesioned region and the image is displayed on a monitor. The operator can perform diagnosis or necessary treatment while watching the image displayed on the monitor.

Furthermore, some endoscope apparatuses are able to perform special-light observation using special light such as infrared light not only for normal observation using white color light but also for observation of blood vessels inside.

In the case of an infrared endoscope apparatus, for example, indocyanine green (ICG) having an absorption peak characteristic in near-infrared light near a wavelength of 805 nm is injected as medicine into the blood of the patient. The object is then irradiated with infrared light near a wavelength of 805 nm and near 930 nm from a light source device by time sharing. A signal of an object image picked up by a CCD is inputted to a processor of the infrared endoscope apparatus. As disclosed, for example, in Japanese Patent Application Laid-Open Publication No.2000-41942, regarding such an infrared endoscope apparatus, there is a proposal on an apparatus whose processor assigns an image near a wavelength of 805 nm to a green color signal (G), an image near a wavelength of 930 nm to a blue color signal (B), and outputs the signals to a monitor. Since the image of infrared light near 805 nm which is more absorbed by the ICG is assigned to the green color, the operator can observe the infrared image with good contrast when ICG is administered.

Furthermore, in recent years, endoscopic submucosal dissection (ESD) using an endoscope to dissect and peel off the submucosa where a lesioned region exists has been widespread. During ESD, in order to prevent the muscularis propria located deeper than the submucosa from being mistakenly perforated or prevent a relatively thick blood vessel in the mucous membrane from being cut, the operator needs to perform treatment such as dissection while checking the positions of the submucosa and the blood vessel.

For this reason, a method of avoiding the risk of mistakenly perforating the muscularis propria may be adopted by dyeing the submucosa with an aqueous solution which is a mixture of a pigment and a physiological saline solution before ESD to make stand out the boundary between the muscularis propria and the submucosa.

However, if a dyeing of submucosa is too thick, visibility of the blood vessel is reduced, which leads to a concern that a blood vessel at a deed part of the mucosa may be severed by mistake. Conversely, if dyeing of submucosa is thinned, the visibility of the blood vessel is improved, but the boundary between the muscularis propria and the submucosa is problematically unclarified.

Accordingly, the present invention was achieved in view of the above-mentioned problems, and an objective of the present invention is to provide endoscope apparatuses with improved visibility at a deed part of the mucosa and visibility of submucosa in ESD.

EP 2 301 418 A1 discloses an endoscope apparatus comprising a light source device, an imaging element and a processor. The light source device includes a plurality of laser light sources. One laser light source LD1 has a central light emission wavelength of 405 nm, two laser light sources each has a central light emission wavelength of 472 nm and two laser light sources each has a central light emission wavelength of 785 nm.

US2009/0118578A1 discloses an endoscope apparatus comprising a light source device emitting narrow band light having a center wavelength of 415nm, 545nm and 615nm. This document discloses all the features of the preamble of claim 1.

### Disclosure of Invention

### Means for Solving the Problem

An endoscope apparatus according to claim 1 is provided.

### Brief Description of the Drawings

Fig. 1 is a configuration diagram illustrating a configuration of an endoscope apparatus according to an embodiment of the present invention;
Fig. 2 is a diagram illustrating an example of ESD according to the embodiment of the present invention;
Fig. 3 is a diagram illustrating output timing of white color light WL emitted from a light source device 4 in a normal-light observation mode according to the embodiment of the present invention;
Fig. 4 is a diagram illustrating output timing of excitation light EX and two narrow-band light beams NBa and NBb emitted from the light source device 4 in an ESD mode according to the embodiment of the present invention;
Fig. 5 is a graph illustrating an intensity distribution DT1 of excitation light EX emitted from an excitation light LED 16b, an intensity distribution DT2 of auto fluorescence FL1 of submucosa 62 by the excitation light and an intensity distribution DT3 of auto fluorescence FL2 of muscularis propria 63 by the excitation light EX according to the embodiment of the present invention;
Fig. 6 is a diagram illustrating a situation of auto fluorescence FL1, FL2 excited by excitation light EX and emitted according to the embodiment of the present invention;
Fig. 7 is a graph illustrating an intensity distribution DT4 of reflected light of narrow-band light NBa near a wavelength of 630 nm emitted from a narrow-band light LED 16c according to the embodiment of the present invention;
Fig. 8 is a diagram illustrating a situation in which narrow-band light NBa is absorbed by a blood vessel and reflected from a tissue within a body cavity other than the blood vessel according to the embodiment of the present invention;
Fig. 9 is a diagram illustrating light absorption spectra of hemoglobin (Hb) and oxygenated hemoglobin (HbO₂) according to the embodiment of the present invention;
Fig. 10 is a graph illustrating an intensity distribution DT5 of narrow-band light NBb near a wavelength of 600 nm emitted from an excitation light LED 16d according to the embodiment of the present invention;
Fig. 11 is a diagram illustrating a situation in which narrow-band light NBb is reflected by the submucosa 62 and the muscularis propria 63 according to the embodiment of the present invention;
Fig. 12 illustrates a light absorption spectrum of indigo carmine according to the embodiment of the present invention;
Fig. 13 is a diagram illustrating a situation in which narrow-band light NBb is reflected by the submucosa 62 and the muscularis propria 63 after the submucosa 62 is removed during ESD according to the embodiment of the present invention;
Fig. 14 is a diagram illustrating a composite image when the submucosa 62 is not removed by an electric knife or the like in the ESD mode according to the embodiment of the present invention;
Fig. 15 is a diagram illustrating a composite image when part of the submucosa 62 is removed by an electric knife or the like in the ESD mode according to the embodiment of the present invention;
Fig. 16 is a diagram illustrating each region of a tissue within a body cavity, and display color and relative intensity of auto fluorescence FL and reflected light of narrow-band light NBa, NBb described in Fig. 5 to Fig. 11;
Fig. 17 is a configuration diagram illustrating a configuration of an endoscope apparatus according to a modification example 1 of the embodiment of the present invention;
Fig. 18 is a diagram illustrating a configuration of the rotation filter 104 according to the modification example 1 of the embodiment of the present invention;
Fig. 19 is a diagram illustrating a configuration of the rotation filter when using a single-color image pickup device according to the modification example 1 of the embodiment of the present invention; and
Fig. 20 is a diagram illustrating an example of a display mode of two images simultaneously displayed on an observation monitor 5 according to a modification example 4 of the embodiment of the present invention.

### Best Mode for Carrying Out the Invention

Hereinafter, embodiments of the present invention will be described with reference to the accompanying drawings.

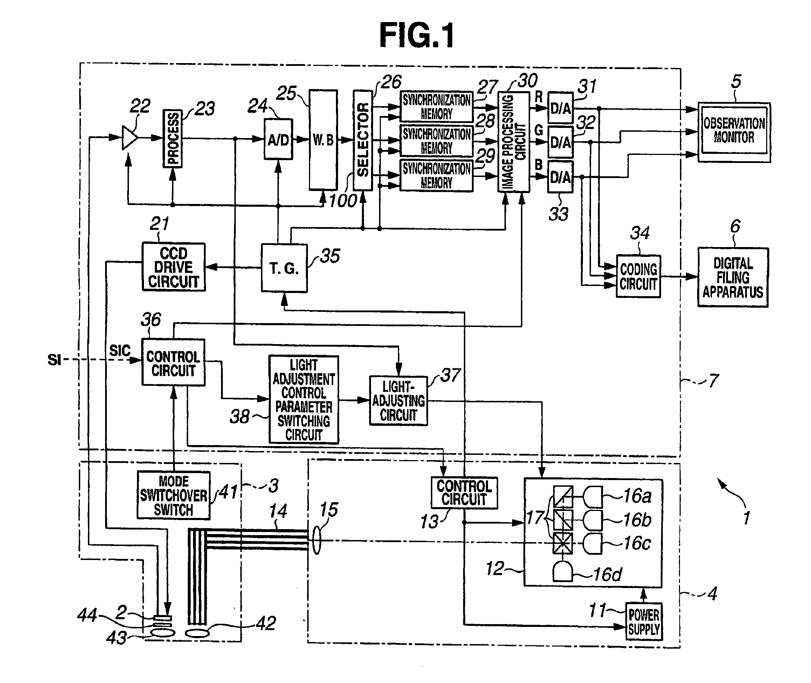

Fig. 1 is a configuration diagram illustrating a configuration of an endoscope apparatus according to an embodiment of the present invention.

As shown in Fig. 1, the endoscope apparatus 1 of the present embodiment is constructed of an electronic endoscope (hereinafter simply referred to as "endoscope") 3 having a CCD 2 which is an image pickup device, a light source device 4 that supplies illuminating light to the endoscope 3, and a video processor (hereinafter referred to as "processor") 7 that applies signal processing to an image pickup signal from the CCD 2 of the endoscope 3, displays the endoscope image on an observation monitor 5, codes and records the endoscope image in a digital filing apparatus 6. An endoscope insertion portion is inserted into a body cavity and the CCD 2 of the endoscope 3 picks up an image of a tissue within the body cavity. The endoscope apparatus 1 has two operation modes; normal-light observation mode and ESD mode. The ESD mode is a mode when ESD is performed. The operator can observe and record an endoscope image in the body cavity, and perform treatment using a treatment instrument while switching between modes using the endoscope apparatus 1.

In the following description, since the normal-light observation mode of the endoscope apparatus 1 is the same as a conventional normal-light observation mode, the configuration of the normal-light observation mode will be described briefly and the ESD mode will be described mainly.

The CCD 2 constitutes an image pickup section or image pickup means that receives reflected light of the illuminating light radiated onto a tissue within a body cavity of an object to be examined and picks up an image of the tissue within the body cavity. The CCD 2 is a color image pickup device with a mosaic primary color filter arranged for each pixel. Instead of the CCD 2, the image pickup device may also be a CMOS image sensor that can perform analog-to-digital (A/D) conversion on a chip.

The light source device 4 is configured by including a power supply 11, an LED illumination section 12 that emits various illuminating light beams as illumination means, a control circuit 13, and a condensing lens 15 that condenses light on the plane of incidence of a light guide 14 disposed inside the endoscope 3. The LED illumination section 12 includes a light-emitting element group including a white color LED 16a, an excitation light LED 16b, a first narrow-band light LED 16c and a second narrow-band light LED 16d, and an optical system 17 such as a half mirror provided in correspondence with each light-emitting element. The optical system 17 condenses light from each LED which is a light-emitting element to the condensing lens 15.

In the present embodiment, the white color LED 16a is a light-emitting element that emits white color light of a wavelength band for normal-light observation.

The excitation light LED 16b is a light-emitting element that emits light near a wavelength of 400 nm as an excitation light EX to excite fluorescence. The excitation light EX is narrow-band light to excite a connective tissue in the submucosa of a tissue within a body cavity as will be described later and enable the connective tissue to emit auto fluorescence.

The narrow-band light LED 16c is a light-emitting element that emits narrow-band light NBa near a wavelength of 630 nm. The narrow-band light NBa is light which is absorbed by a blood vessel to draw images of a relatively thick blood vessel running through the submucosa and muscularis propria as will be described later. The narrow-band light NBa is light of a wavelength band on the longer wavelength side than the narrow-band light NBb and less susceptible to influences of scattering or absorption on the surface of a tissue within a body cavity.

The narrow-band light LED 16d is a light-emitting element that emits narrow-band light NBb near a wavelength of 600 nm. The narrow-band light NBb is light absorbed by indigo carmine which is a substance locally injected into the submucosa as will be described later. In particular, the narrow-band light NBb is narrow-band light corresponding to an absorption peak in a light absorption characteristic of indigo carmine.

Here, in the case of light near a wavelength of 630 nm, "near" means that narrow-band light has a central wavelength of 630 nm and has a range of wavelengths on the order of a full width at half maximum ±10 nm centered on the wavelength of 630 nm. The same applies to other wavelengths such as wavelength 600 nm and wavelength 550 nm which will be described later.

Therefore, the LED illumination section 12 of the light source device 4 constitutes illumination means or an illumination section that can switch between excitation light EX, narrow-band light NBa and NBb, and radiate the selected light. Furthermore, the light source device 4 is an illumination section that can illuminate an object with not only excitation light EX, narrow-band light NBa and NBb, but also white color light.

The control circuit 13 controls the power supply 11 and the LED illumination section 12 so that the light source device 4 emits illuminating light in accordance with each operating mode.

The processor 7 is configured by including a CCD drive circuit 21 which is a CCD driver, an amplifier 22, a process circuit 23, an analog digital (A/D) converter 24, a white balance circuit (hereinafter referred to as "W.B") 25, a selector 26, synchronization memories 27, 28 and 29, an image processing circuit 30, digital analog (D/A) converters 31, 32 and 33, a coding circuit 34, a timing generator (hereinafter referred to as "T.G") 35, a control circuit 36, a light-adjusting circuit 37 and a light adjustment control parameter switching circuit 38.

The endoscope 3 includes, in addition to the aforementioned CCD 2 and the light guide 14, a mode switchover switch 41, a lens 42 which is an optical system for illuminating light, a lens 43 which is an objective optical system for the CCD 2 and an excitation light cut filter 44. The light guide 14 is light guiding means for guiding the illuminating light emitted from the light source device 4 into a living tissue in the body cavity of an observation object. The lens 43 condenses fluorescence or reflected light from the tissue within the body cavity to the CCD 2 and the CCD 2 picks up an image of the tissue within the body cavity, which is the object, using the condensed light and outputs an image pickup signal. The excitation light cut filter 44 is a filter to intercept excitation light and has a passband for light having a wavelength of 430 to 690 nm.

The CCD drive circuit 21 outputs a drive signal to drive the CCD 2 provided in the endoscope 3. The CCD 2 is a color CCD that is driven by the drive signal and outputs an image pickup signal. Furthermore, the amplifier 22 is intended to amplify the image pickup signal of the image of the tissue within the body cavity picked up by the CCD 2 via the objective optical system 43 provided at a distal end of the endoscope 3.

The process circuit 23 performs correlation double sampling and noise reduction or the like on the image pickup signal via the amplifier 22. The A/D converter 24 converts the analog image pickup signal that has passed through the process circuit 23 to a digital image signal.

The W.B 25 performs gain adjustment and white balance processing on the image signal digitized by the A/D converter 24 so that the R signal of the image signal has brightness equal to that of the B signal of the image signal with reference to the G signal of the image signal, for example.

The selector 26 classifies the image signal from the W.B 25 by color signal, that is, by wavelength and outputs the classified image signals. The image signals classified by color from the selector 26 are supplied to the synchronization memories 27, 28 and 29, each of which stores image signals.

The image processing circuit 30 reads the respective image signals stored in the synchronization memories 27, 28 and 29 and performs amplification processing, moving image color shift correction processing or the like. In a normal-light observation mode, one of the synchronization memories 27, 28 and 29 is used, whereas in an ESD mode, the synchronization memories 27, 28 and 29 are used. Image signals of fluorescence and two narrow band reflected light beams are stored in the synchronization memories 27, 28 and 29 respectively.

In the normal-light observation mode, the image processing circuit 30 outputs the respective image signals of RGB to their corresponding channels of RGB to display an object image with a predetermined color image. In the ESD mode, since the respective image signals of the fluorescence image and two narrow-band images are assigned to any one of the RGB channels beforehand, the image processing circuit 30 outputs the respective image signals generated to their corresponding channels of RGB.

Thus, the synchronization memories 27, 28 and 29 and the image processing circuit 30 constitute image signal generating means or an image signal generation section that generates an image signal from the signal picked up through the CCD 2 and outputs the image signal to the observation monitor 5.

The D/A converters 31, 32 and 33 convert the image signal from the image processing circuit 30 which is the image signal generation section to an analog video signal and output the image signal to the observation monitor 5 as a display apparatus. Furthermore, the outputs of the D/A converters 31, 32 and 33 are supplied to the coding circuit 34 and the digital filing apparatus 6 records endoscope images. Outputting to the observation monitor 5 and outputting to the digital filing apparatus 6 are performed according to the operator's operation on an operation panel (not shown) of the processor 7. Thus, the D/A converters 31, 32 and 33 and the observation monitor 5 constitute display means or a display section that synthesizes a plurality of image signals generated in the image signal processing circuit 30 and displays the composite image.

The T.G 35 is a circuit for controlling timing of illumination and image pickup. For this purpose, the T.G 35 receives a synchronization signal synchronized with the drive of each LED of the LED illumination section 12 from the control circuit 13 of the light source device 4 as input and outputs various timing signals to the respective circuits in the above-described processor 7.

The light-adjusting circuit 37 is a circuit that generates a light adjustment signal to adjust the amount of light emission of each LED of the LED illumination section 12 based on a light adjustment control parameter from the light adjustment control parameter switching circuit 38. The light adjustment control parameter switching circuit 38 is a circuit that switches between light adjustment control parameters to be outputted to the light-adjusting circuit 37 based on a control signal from the control circuit 36.

The light-adjusting circuit 37 is designed to control the LED illumination section 12 of the light source device 4 and perform appropriate brightness control based on the light adjustment control parameters from the light adjustment control parameter switching circuit 38 and the image pickup signal that has passed through the process circuit 23.

Furthermore, the endoscope 2 is provided with the mode switchover switch 41 for switching between the normal-light observation mode and ESD mode, and the output of the mode switchover switch 41 is outputted to the control circuit 36 in the processor 7. The control circuit 36 of the processor 7 is designed to output a control signal to the light adjustment control parameter switching circuit 38.

Each circuit in the processor 7 executes predetermined processing in accordance with a specified mode. Processing corresponding to the normal-light observation mode and the ESD mode is executed respectively and the observation monitor 5 displays a normal-light observation mode or ESD image.

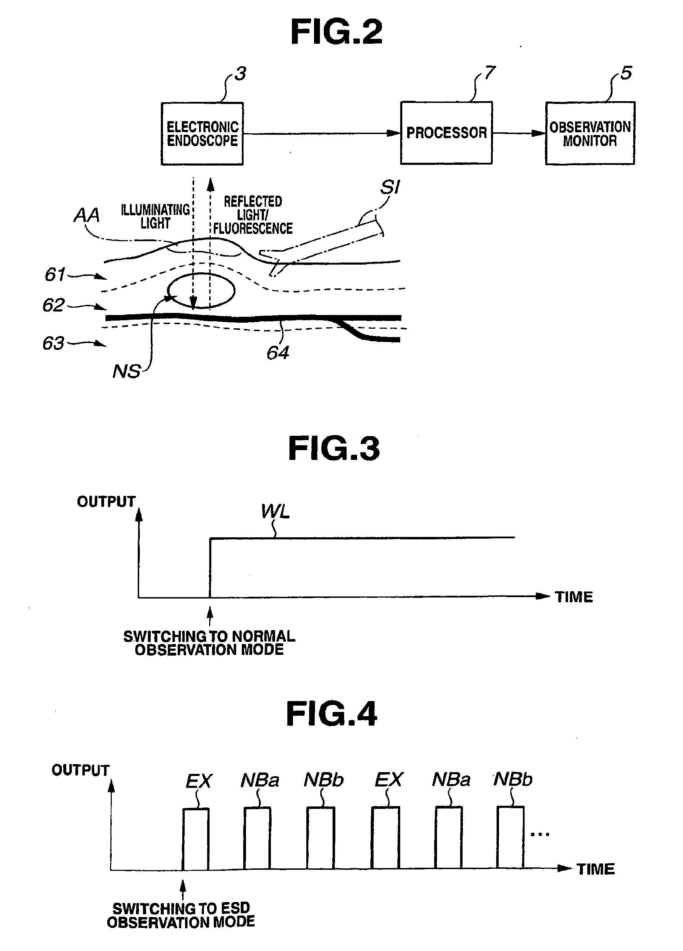

Fig. 2 is a diagram illustrating an example of ESD. The operator can insert the insertion portion of the endoscope 3 into the body cavity, locate the distal end portion of the endoscope insertion portion in the vicinity of a lesioned region and perform normal-light observation of a tissue within a body cavity using normal light in the normal-light observation mode. When performing ESD treatment, the operator operates the mode switchover switch 41 to change the mode of the endoscope apparatus 1 to the ESD mode. As will be described later, in the normal-light observation mode, the white color LED 16a remains on continuously, whereas in the ESD mode, the LEDs 16b, 16c and 16d repeatedly turn on in predetermined order.

During ESD, a physiological saline solution NS containing a pigment such as indigo carmine is locally injected into a submucosa 62 below a mucosal layer 61. A blood vessel 64 is a relatively thick blood vessel of 1 to 2 mm in diameter running through the submucosa 62 or muscularis propria 63. A lesioned region AA of the mucosal layer 61 is raised by the local injection of the physiological saline solution NS into the submucosa 62. With the lesioned region AA being raised, the operator dissects the submucosa 62 below the lesioned region AA using a treatment instrument SI such as an electric knife and peels off the mucosal layer 61 including the lesioned region AA.

In the ESD mode, the control circuit 36 of the endoscope apparatus 1 sequentially drives the excitation light LED 16b, two narrow-band light LEDs 16c and 16d in the LED illumination section 12 of the light source device 4 and supplies a control signal to the control circuit 13 so as to sequentially and repeatedly emit the excitation light EX, two narrow-band light beams NBa and NBb from the light source device 4. Furthermore, the control circuit 36 controls the image processing circuit 30 in the processor 7 so as to perform image processing for the ESD mode.

Fig. 3 is a diagram illustrating output timing of white color light WL emitted from the light source device 4 in the normal-light observation mode. Fig. 4 is a diagram illustrating output timing of the excitation light EX and two narrow-band light beams NBa and NBb emitted from the light source device 4 in the ESD mode.

When the mode switchover switch 41 is operated and the normal-light observation mode is set, the white color LED 16a is driven and white color light WL is continuously emitted as shown in Fig. 3. The white color light WL is radiated onto the tissue within the body cavity via the light guide 14 and an image thereof is picked up by the CCD 2 which is a color image pickup device. The image pickup signal of the CCD 2 is supplied to the processor 7, subjected to predetermined image processing and an endoscope image in normal-light observation is displayed on the observation monitor 5. Thus, when the normal-light observation mode is set, the operator causes the observation monitor 5 to display the image generated by the reflected light obtained by radiating the white color light WL onto the tissue within the body cavity, and can thereby observe the tissue within the body cavity.

When the ESD mode is set, the excitation light LED 16b, the first narrow-band light LED 16c and the second narrow-band light LED 16d are sequentially driven, and as shown in Fig. 4, the excitation light EX, the narrow-band light NBa and the narrow-band light NBb are successively emitted in that order. The CCD 2 receives auto fluorescence from the tissue within the body cavity at irradiation timing of the excitation light EX and receives reflected light of the narrow-band light NBa from the tissue within the body cavity at irradiation timing of the narrow-band light NBa, and receives reflected light of the narrow-band light NBb from the tissue within the body cavity at irradiation timing of the narrow-band light NBb.

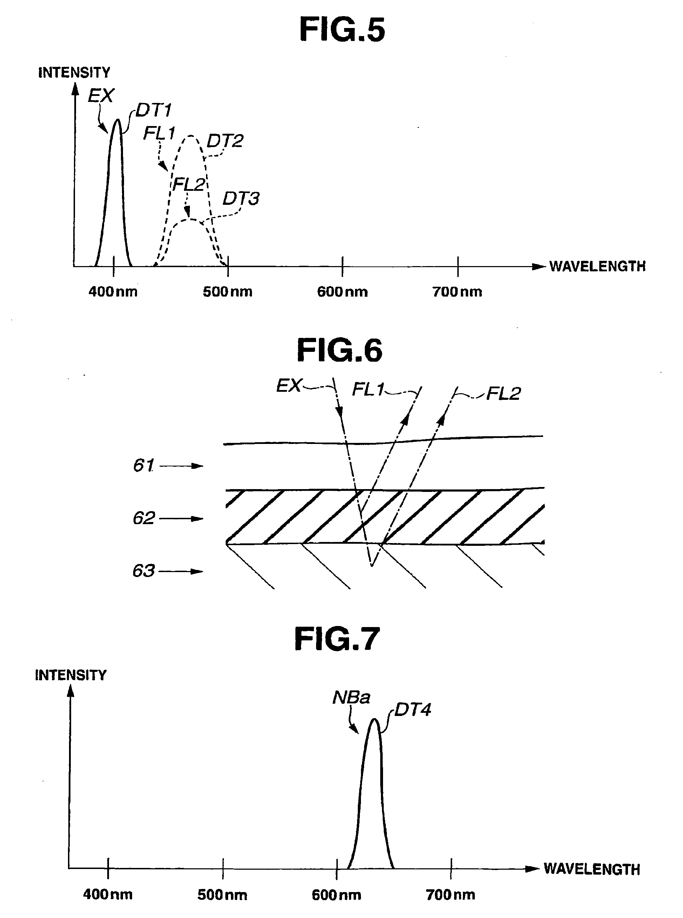

Here, intensities of the excitation light EX and two narrow-band light beams NBa and NBb emitted from the light source 4 in the ESD mode will be described. Fig. 5 is a graph illustrating an intensity distribution DT1 (solid line) of the excitation light EX emitted from the excitation light LED 16b, an intensity distribution DT2 (dotted line) of auto fluorescence FL1 of the submucosa 62 by the excitation light thereof, and an intensity distribution DT3 (dotted line) of auto fluorescence FL2 of the muscularis propria 63 by the excitation light EX thereof. Hereinafter, two auto fluoresce FL1 and FL2 beams will be collectively referred to as "auto fluorescence FL". The auto fluorescence FL passes through a blue (B) color filter of the CCD 2 which is a color image pickup device and is received by the CCD 2.

Fig. 6 is a diagram illustrating a situation of auto fluorescence FL1, FL2 excited by the excitation light EX and emitted. The auto fluorescence FL is light having a wavelength range of 430 to 500 nm. As shown in Fig. 5 and Fig. 6, regarding the intensity of auto fluorescence generated from the excitation light EX near a wavelength of 400 nm, the auto fluorescence FL1 from the submucosa 62 is stronger than the auto fluorescence FL2 from the muscularis propria 63. The auto fluorescence FL is light generated when a connective tissue in the tissue within a body cavity, for example, fibrous tissue such as collagen is excited by the excitation light EX. Fluorescence of collagen which is dominant in the connective tissue 62 has high excitation efficiency in a near-ultraviolet to violet band. In Fig. 6, the submucosa 62 is shown by thick diagonal lines and the muscularis propria 63 is shown by thin diagonal lines indicating that the auto fluorescence FL1 of the submucosa 62 has stronger intensity than the auto fluorescence FL2 of the muscularis propria 63.

Fig. 7 is a graph illustrating an intensity distribution DT4 of reflected light of the narrow-band light NBa near a wavelength of 630 nm emitted from the narrow-band light LED 16c. The reflected light of the narrow-band light NBa near the wavelength of 630 nm passes through a red (R) color filter of the CCD 2 which is a color image pickup device and is received by the CCD 2.

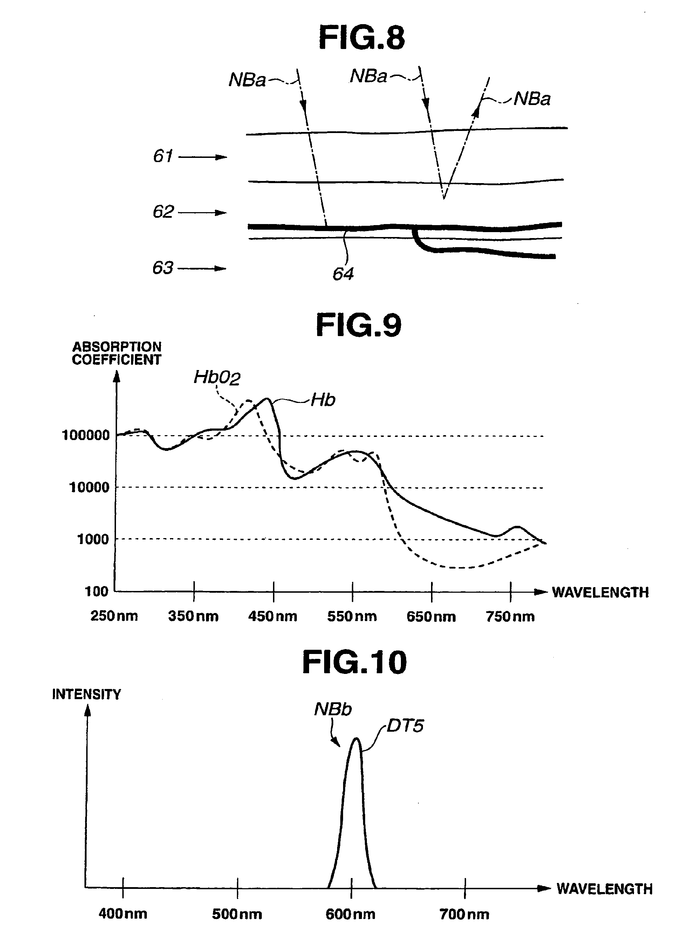

Fig. 8 is a diagram illustrating a situation in which the narrow-band light NBa is absorbed by a blood vessel and reflected by a tissue within a body cavity other than the blood vessel. The narrow-band light NBa near a wavelength of 630 nm is less susceptible to influences of light scattering by the tissue within a body cavity than the blue color narrow-band light for emphasizing the capillary vessel of the surface layer. As shown in Fig. 8, most of the narrow-band light NBa near a wavelength of 630 nm is absorbed and not reflected by a relatively thick blood vessel 64 running through the submucosa 62 or muscularis propria 63 and reflected by tissues within the body cavity other than the blood vessel. This is because the narrow-band light NBa near 630 nm has a characteristic of being absorbed relatively more due to hemoglobin in the blood. Thus, the narrow-band light NBa is used to draw images of such a blood vessel.

Fig. 9 is a diagram illustrating light absorption spectra of hemoglobin (Hb) and oxygenated hemoglobin (HbO₂). In Fig. 9, a solid line shows a light absorption spectrum of hemoglobin (Hb) and a dotted line shows a light absorption spectrum of oxygenated hemoglobin (HbO₂).

Fig. 10 is a graph illustrating an intensity distribution DT5 of the narrow-band light NBb near a wavelength of 600 nm emitted from the excitation light LED 16d. The reflected light of the narrow-band light NBb near the wavelength of 600 nm passes through the R (red) color filter of the CCD 2 which is a color image pickup device and is received by the CCD 2.

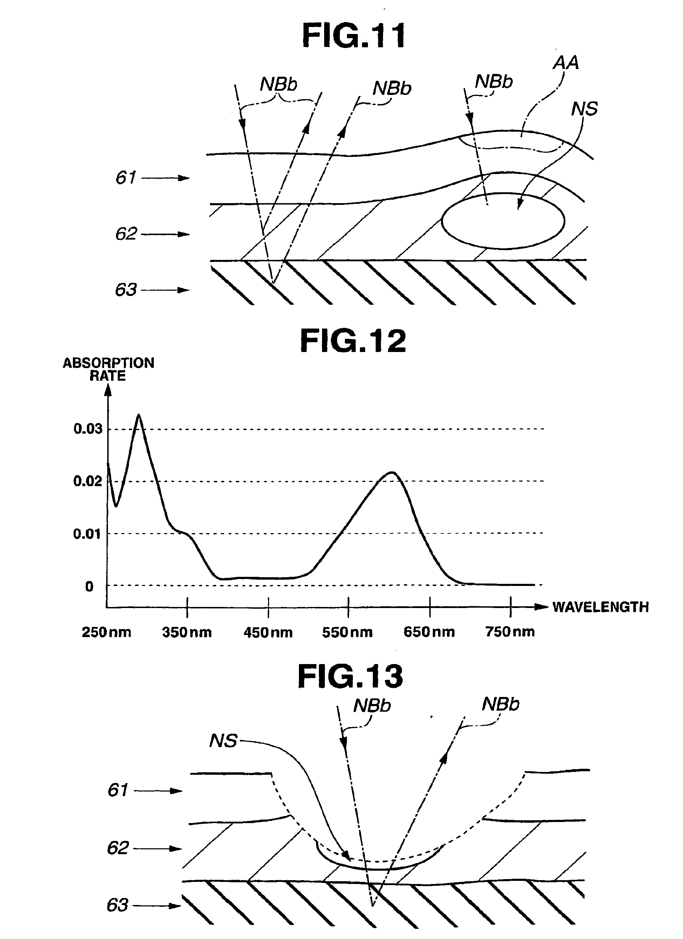

Fig. 11 is a diagram illustrating a situation in which the narrow-band light NBb is reflected by the submucosa 62 and the muscularis propria 63. The narrow-band light NBb near the wavelength of 600 nm is light of a wavelength band strongly absorbed by indigo carmine which is a pigment. As shown in Fig. 11, regarding the intensity of the reflected light of the narrow-band light NBb near the wavelength of 600 nm, the light from the muscularis propria 63 is stronger than the light from the submucosa 62. In Fig. 11, the muscularis propria 63 is shown by thick diagonal lines and the submucosa 62 is shown by thin diagonal lines indicating that the intensity of the reflected light of the muscularis propria 63 is stronger than that of the reflected light of the submucosa 62.

During ESD, a physiological saline solution NS containing a pigment substance is locally injected into the submucosa 62 below the mucosal layer 61 where the lesioned region exists. Here, indigo carmine is used as the pigment.

Fig. 12 illustrates a light absorption spectrum of indigo carmine. As shown in Fig. 12, indigo carmine has an absorption peak characteristic of absorbing the narrow-band light NBb near a wavelength of 600 nm in particular. Therefore, before the submucosa 62 is removed during ESD, the narrow-band light NBb is more likely to be absorbed by indigo carmine in the physiological saline solution NS as shown in Fig. 11.

Fig. 13 is a diagram illustrating a situation in which after the submucosa 62 is removed during ESD, the narrow-band light NBb is reflected by the submucosa 62 and the muscularis propria 63. As shown by a dotted line in Fig. 13, when the submucosa 62 is removed, the amount of indigo carmine in the physiological saline solution NS decreases, and therefore the rate of absorption by indigo carmine of the narrow-band light NBb decreases and the amount of reflected light of the narrow-band light NBb in the submucosa 62 and the muscularis propria 63 increases.

The auto fluorescence FL, and the reflected light beams of the narrow-band light NBa, NBb are received by the CCD 2. The CCD 2 outputs the respective image pickup signals of light and the respective image pickup signals are supplied to the selector 26 via the amplifier 22 or the like. The selector 26 stores the image of the auto fluorescence FL, the two images of the two reflected light beams of the narrow-band light NBa, NBb in the synchronization memories 27, 28 and 29 respectively in accordance with predetermined timing from the T.G 35. The respective images stored in the synchronization memories 27, 28 and 29 are divided into their respective RGB channels by the image processing circuit 30, then synthesized and outputted to the observation monitor 5.

The auto fluorescence FL and the reflected light beams of the respective narrow-band light NBa, NBb are generated using signals of pixels of specific color filters of the color filters of the CCD 2, and therefore the image processing circuit 30 performs interpolation processing for interpolation between necessary pixels.

As shown above, in the ESD mode, the operator can view a tissue within a body cavity with a composite image of the auto fluorescence FL and the reflected light beams of the narrow-band light NBa, NBb obtained by irradiating the tissue within the body cavity with the excitation light EX and two narrow-band light beams NBa and NBb.

Here, descriptions will be given about display states of an image of a tissue within a body cavity when the submucosa 62 is not removed as shown in Fig. 11 in the ESD mode and an image of a tissue within a body cavity when the submucosa 62 is removed as shown in Fig. 13.

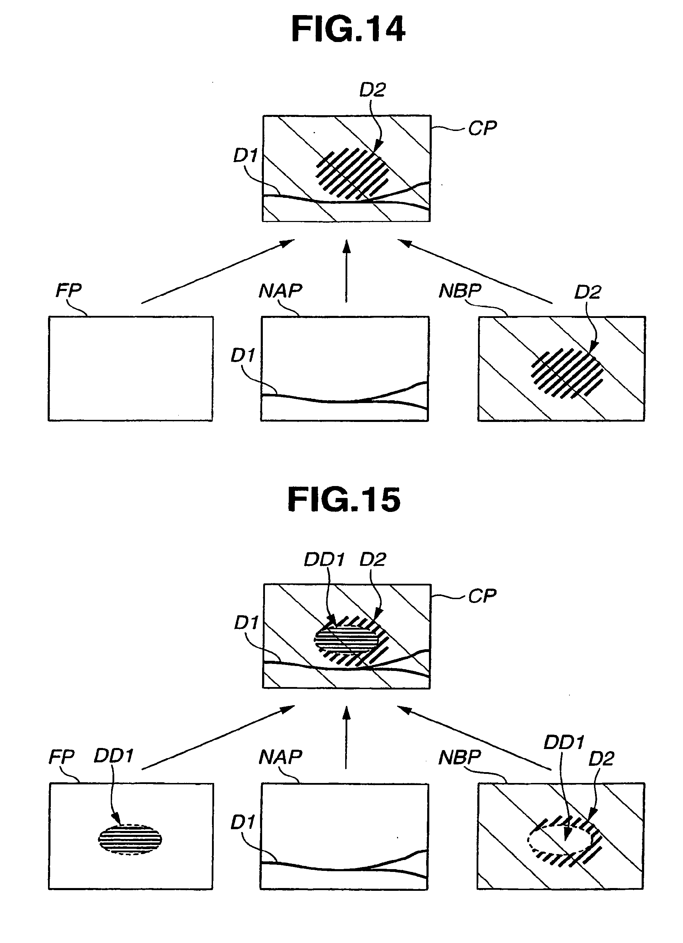

Fig. 14 is a diagram illustrating a composite image when the submucosa 62 is not removed using an electric knife or the like in the ESD mode (case as shown in Fig. 11).

In the ESD mode, in the case as shown in Fig. 11, by irradiating the mucosal layer 61 with the excitation light EX, two narrow-band light beams NBa and NBb, the image processing circuit 30 acquires the fluorescence images FP by the auto fluorescence FL1, FL2 of the submucosa 62 and the muscularis propria 63, the narrow-band image NAP by the reflected light of the narrow-band light NBa and the narrow-band image NBP by the reflected light of the narrow-band light NBb. The image signals of the three images FP, NAP and NBP are assigned to three channels of R, B and G respectively and synthesized.

As shown in Fig. 14, since the auto fluorescence by the submucosa 62 is strong, the fluorescence image FP is a bright image as a whole. Furthermore, regarding the narrow-band image NAP, a portion D1 where the narrow-band light NBa is absorbed by the blood vessel 64 is shown as a dark image. Regarding the narrow-band image NBP, a portion D2 where the narrow-band light NBb is absorbed by the physiological saline solution NS in the submucosa 62 (to be exact, indigo carmine) is shown as a dark image. These three images FP, NAP and NBP are synthesized and a composite image CP is displayed on the observation monitor 5.

Fig. 15 is a diagram illustrating a composite image when part of the submucosa 62 is removed using an electric knife or the like in the ESD mode (case as shown in Fig. 13).

In the case as shown in Fig. 13, when the excitation light EX and two narrow-band light beams NBa and NBb are radiated, since the auto fluorescence FL1 of part of the removed submucosa 62 becomes weak, a removed portion DD1 of the submucosa 62 is shown as a dark image in the fluorescence image FP. Furthermore, a portion D1 where the narrow-band light NBa is absorbed by the blood vessel 64 is shown as a dark image in the narrow-band image NAP. Since the amount of the narrow-band light NBb absorbed by the physiological saline solution NS (to be exact, indigo carmine) in the submucosa 62 decreases, the narrow-band image NBP is an image where the reflected light from the muscularis propria 63 is strong in the removed portion DD1.

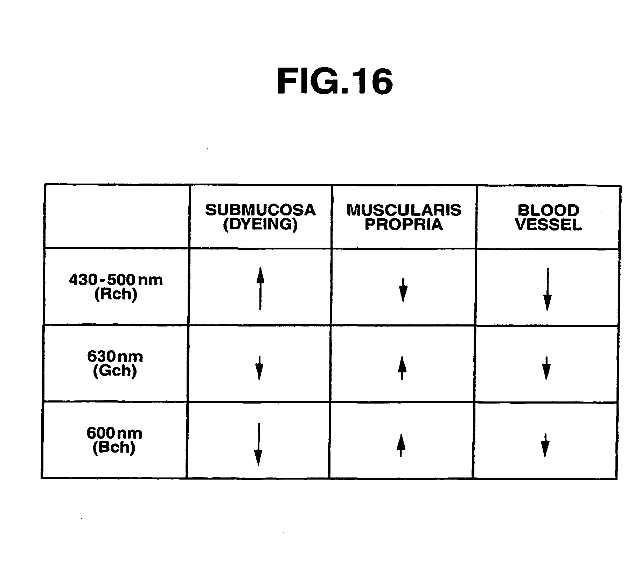

Fig. 16 is a diagram illustrating each region of a tissue within a body cavity, and display color and relative intensity of auto fluorescence FL and reflected light of narrow-band light NBa, NBb described in Fig. 5 to Fig. 11. As shown in Fig. 16, in the present embodiment, the auto fluorescence FL (wavelength 430 to 500 nm) is assigned to the red (R) channel, the reflected light (near a wavelength of 630 nm) of the narrow-band light NBa is assigned to the green (G) channel, and the reflected light (near a wavelength of 600 nm) of the narrow-band light NBb is assigned to the blue (B) channel. Thus, the submucosa 62 is displayed in a red to orange color, the muscularis propria 63 is displayed in a light cyan color and the blood vessel 64 is displayed in a black to dark blue color.

Fig. 16 shows that the intensity of the auto fluorescence FL (wavelength 430 to 500 nm) is strong in the submucosa 62 containing indigo carmine, small in the muscularis propria 63 and smaller in the blood vessel 64. Fig. 16 shows that the intensity of the reflected light (near a wavelength of 630 nm) of the narrow-band light NBa is smaller in the submucosa 62 and the blood vessel 64 than the muscularis propria 63. Furthermore, Fig. 16 shows that the intensity of the reflected light (near a wavelength of 600 nm) of the narrow-band light NBb is smaller in the blood vessel 64 than the muscularis propria 63, and is much smaller in the submucosa 62 containing indigo carmine.

Through the aforementioned assignment of the three image signals of FP, NAP and NBP to the three channels of R, B and G, in the composite image CP, the color tone of the tissue within a body cavity in the lower part changes from red to cyan as the observation point approximates from the submucosa 62 to the muscularis propria 63, and the relatively thick blood vessel 64 existing in the submucosa 62 or muscularis propria 63 is displayed in dark blue against the surrounding tissue within a body cavity which is drawn in red.

Here, although the auto fluorescence FL, narrow-band light NBa and narrow-band light NBb are assigned to the R, G and B channels respectively, other assignment methods may also be adopted. For example, the auto fluorescence FL, narrow-band light NBa, and narrow-band light NBb may be assigned to the G, B and R channels respectively.

This is because, the auto fluorescence FL emits strong fluorescence mainly in the submucosa 62, the narrow-band light NBb near a wavelength of 600 nm is more likely to be absorbed by indigo carmine, and therefore the reflected light thereof is weak. Furthermore, since the narrow-band light NBa near a wavelength of 630 nm is absorbed by the relatively thick blood vessel 64, the reflected light thereof is weaker and the submucosa 62, the muscularis propria 63 and the blood vessel 64 are drawn with different color tone differences as shown in Fig. 16.

Therefore, when a comparison is made between Figs. 14 and 15, when part of the submucosa 62 is removed and the removed portion DD1 approaches the muscularis propria 63 during ESD, the removed portion DD1 of the submucosa 62 changes from the color of the fluorescence image FP (red-based) to the color of the portion DD1 of the narrow-band image NBP (blue-based). That is, as the removal of the submucosa 62 advances during ESD, the color of the removed portion changes to a different color, and therefore the operator can easily recognize that the removed portion DD1 approaches the muscularis propria 63.

Furthermore, the narrow-band light NBa near a wavelength of 630 nm is used as light to increase visibility of the blood vessel and reduce the risk of hemorrhage and further the narrow-band light NBb near a wavelength of 600 nm increases the contrast between the submucosa 62 dyed with indigo carmine and the muscularis propria 63 therebelow, and therefore the physiological saline solution NS having a small indigo carmine content may be used.

In the aforementioned embodiment, the pigment contained in the physiological saline solution NS which is injected into the submucosa 62 is indigo carmine, but the pigment may be another pigment, for example, methylene blue. In that case, narrow-band light NBb that matches the light absorption spectrum characteristic of the pigment is selected.

As described above, according to the aforementioned embodiment, it is possible to realize an endoscope apparatus with improved visibility of blood vessels in the depth of the mucous membrane and improved visibility of the submucosa during ESD.

Next, modification examples of the aforementioned embodiment will be described.

### (Modification example 1)

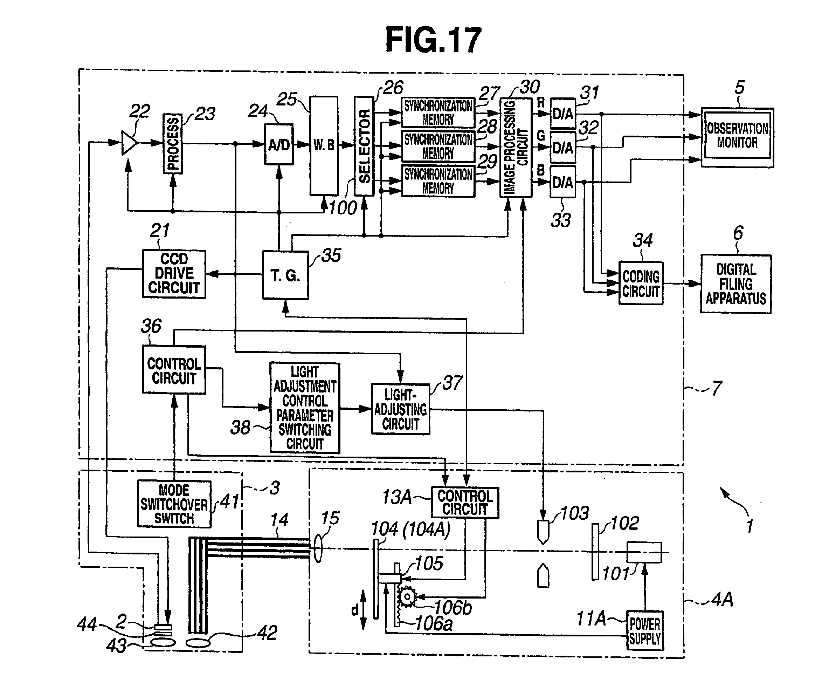

The present modification example is a modification example of the light source device. Fig. 17 is a configuration diagram illustrating a configuration of an endoscope apparatus according to the present modification example. Fig. 17 is different from Fig. 1 in the configuration of the light source device. In Fig. 17, the same components as those in Fig. 1 are assigned the same reference numerals and descriptions thereof will be omitted.

A light source device 4A is configured by including a xenon lamp 101 that emits illuminating light (white color light) as illumination means, a heat radiation cut filter 102 that intercepts heat radiation of the white color light, a diaphragm apparatus 103 that controls a light quantity of the white color light via the heat radiation cut filter 102, a rotation filter 104 that transforms illuminating light into frame sequential light as band limiting means, and a control circuit 13A that controls the rotation and position of the rotation filter 104. The frame sequential light that has passed through the rotation filter 104 is condensed by the condensing lens 15 onto the plane of incidence of the light guide 14 disposed in the electronic endoscope 3. The xenon lamp 101, the rotation filter 104 and the light guide 14 constitute an irradiation section or irradiating means that illuminates an object to be examined with illuminating light.

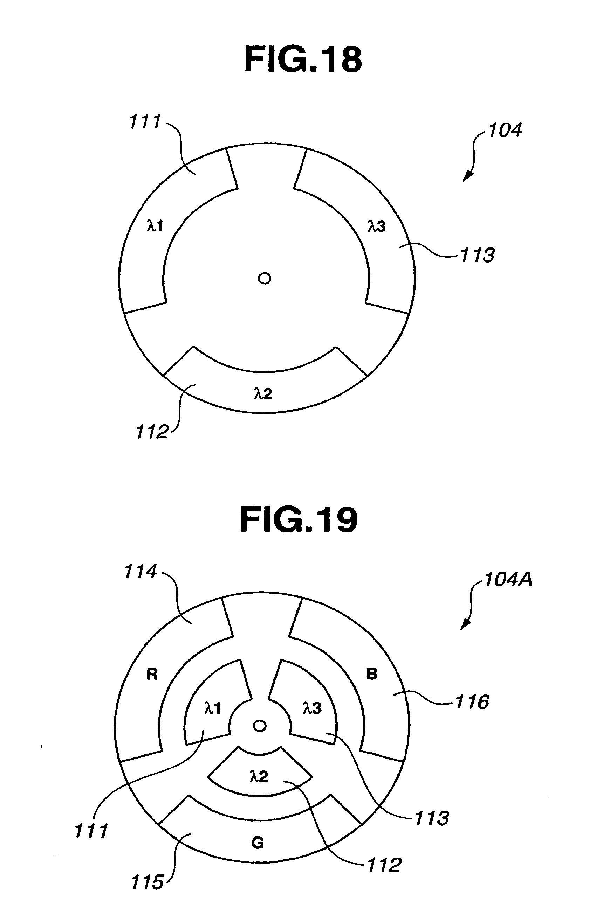

Fig. 18 is a diagram illustrating a configuration of the rotation filter 104. The rotation filter 104 as a wavelength band limiting section or wavelength band limiting means is configured in a disk shape as shown in Fig. 18, having a structure, the center of which corresponds to the axis of rotation. The rotation filter 104 is provided with three filters 111, 112 and 113 in a circumferential direction to allow light beams having three predetermined narrow-band wavelengths to pass therethrough.

The filter 111 is a filter that allows light near a wavelength of 400 nm (=λ1) to pass therethrough as excitation light EX. The filter 112 is a filter that allows light near a wavelength of 630 nm (=λ2) to pass therethrough as narrow-band light NBa. The filter 113 is a filter that allows light near a wavelength of 600 nm (=λ3) to pass therethrough as narrow-band light NBb.

The rotation filter 104 is disposed on an optical path from the xenon lamp 101 which is the light emerging section of illuminating light to the image pickup surface of the CCD 2 and limits the illuminating light so as to narrow the illuminating light having a plurality of wavelength bands to light having at least three wavelength bands. The control circuit 13A controls the motor 105 for rotating the rotation filter 104 and thereby controls the rotation of the rotation filter 104.

A rack 106a is connected to the motor 105 and a motor (not shown) is connected to a pinion 106b and the rack 106a is attached to the pinion 106b so as to be screwed together. The control circuit 13A controls the rotation of the motor connected to the pinion 106b to move the rotation filter 104 in a direction shown by an arrow d, and can thereby change the position of the rotation filter 104.

Power is supplied to the xenon lamp 101, the diaphragm apparatus 103, the rotation filter motor 105, and the motor (not shown) connected to the pinion 19b from a power supply 11A.

When the operator operates the mode switchover switch 41 to select a normal-light observation mode, the rotation filter 104 moves out of the optical path, white color light emitted through the heat radiation cut filter 102 impinges on the lens 15 and the white color light is introduced into the light guide 14. Furthermore, when the operator operates the mode switchover switch 41 to select an ESD mode, the rotation filter 104 is disposed on the optical path and the excitation light EX and two narrow-band light beams NBa and NBb that have passed through the filters 111, 112 and 113 are sequentially introduced into the light guide 14 under the control of the control circuit 13A.

Therefore, operations similar to those of the aforementioned embodiment can also be obtained using the light source device 4A as shown in Fig. 17.

In the aforementioned modification example, a color image pickup device is used as the image pickup device, but the image pickup device may also be a single-color image pickup device. In such a case, a rotation filter 104A has a configuration as shown in Fig. 19. Fig. 19 is a diagram illustrating a configuration of a rotation filter when using a single-color image pickup device.

On the outer circumference side of the rotation filter 104A, an R (red) filter section 114, a G (green) filter section 115, and a B (blue) filter section 116 constituting a set of filters to output frame sequential light having a spectral characteristic for normal-light observation are arranged along the circumferential direction as a first filter group. Furthermore, on the inner circumference side of the rotation filter 104A, three filters 111, 112 and 113 that allow light of three predetermined narrow-band wavelengths to pass therethrough are arranged along the circumferential direction as a second filter group.

The control circuit 13A controls the rotation of the motor connected to the pinion 106b so that the first filter group is disposed on the optical path in the normal-light observation mode, whereas in the ESD mode, the second filter group is disposed on the optical path.

RGB image signals from the CCD are stored in the three synchronization memories 27, 28 and 29 in synchronization with the rotation of the rotation filter 104A. That is, in the normal-light observation mode, the three RGB image signals are stored in the synchronization memories 27, 28 and 29 respectively, and in the ESD mode, the auto fluorescence FL and reflected light of the two narrow-band light beams NBa and NBb are stored in the synchronization memories 27, 28 and 29 respectively.

As described above, the configuration shown in the present modification example can also obtain operations and effects similar to those of the aforementioned embodiment.

### (Modification example 2)

In the aforementioned embodiment and modification example, the narrow-band light NBa near a wavelength of 630 nm is used to draw images of a relatively thick blood vessel, but when images of a finer blood vessel are preferred to be drawn, the narrow-band light NBc near a wavelength of 550 nm may be used. That is, the narrow-band light NBc near the wavelength of 550 nm is light of a wavelength band on the shorter wavelength side than the narrow-band light NBb near a wavelength of 600 nm and less susceptible to influences of scattering or absorption on the surface of a tissue within a body cavity.

The narrow-band light NBc near the wavelength of 550 nm has an advantage of being less susceptible to influences of indigo carmine and capable of drawing images of finer blood vessels.

As described above, the configuration shown in the present modification example can also obtain operations and effects similar to those of the aforementioned embodiment.

### (Modification example 3)

In the aforementioned embodiment, a composite image CP including a fluorescence image FP is displayed in the ESD mode, but the fluorescence image FP may not be displayed in the composite image CP in the ESD mode.

In the present modification example, the ESD mode further includes a fluorescence image display mode FM in which the fluorescence image FP is displayed in the composite image CP and a fluorescence image non-display mode NFM in which the fluorescence image FP is not displayed in the composite image CP.

In ESD, when a spark or the like occurs with the use of an electric knife or the like, a smear or halation may occur in an observed image displayed on the observation monitor 5. Since the auto fluorescence FL is originally weak light, the amplification rate of the auto fluorescence FL is high and when halation or the like occurs, the observed image displayed on the observation monitor 5 may be distorted considerably, making observation impossible.

Thus, in the ESD mode, when an output signal of a treatment instrument such as an electric knife is in on-state (case 1) or when a fluorescence image non-display mode NFM is selected by a predetermined operation switch (case 2) or when halation or the like is detected (case 3), the processor 7 controls the synthesis of images so as to select the fluorescence image non-display mode NFM in which the fluorescence image FP is not included in the composite image CP.

In case 1, as shown by a dotted line in Fig. 1, an output signal SIC of a surgical treatment instrument SI such as an electric knife is inputted to the control circuit 36 so that any one of the fluorescence image display mode FM and fluorescence image non-display mode NFM is automatically selected in accordance with the output state of the electric knife or the like. Thus, when there is an output from the surgical treatment instrument, the fluorescence image non-display mode NFM is selected and it is thereby possible to suppress the occurrence of halation or the like on the screen.

In case 2, one of the group of switches of the mode switchover switch 41 is a switch to select the fluorescence image display mode FM or fluorescence image non-display mode NFM, and the fluorescence image non-display mode NFM is selected according to the setting of the operator. Therefore, the fluorescence image non-display mode NFM is selected in accordance with a selection or an instruction of the operator and the occurrence of halation or the like on the screen can be suppressed.

In case 3, as shown by the dotted line in Fig. 1, the image processing circuit 30 is provided with, for example, a halation determination circuit 30a. The halation determination circuit 30a is a halation detection section that detects a halation state included in the fluorescence image FL based on the number of saturation pixels in the fluorescence image FL. When the halation determination circuit 30a detects halation, the image processing circuit 30 instructs that the fluorescence image FP should not be displayed in the composite image CP and the fluorescence image non-display mode NFM is automatically selected. Detection of halation can be performed, for example, by judging whether or not at least a predetermined number of pixels among all pixels of the image have luminance equal to or above a predetermined threshold.

As described above, the instruction of the fluorescence image non-display mode NFM is an instruction for not displaying the fluorescence image FL and that instruction is given based on the operation state of a predetermined switch, output state of the treatment instrument or state of halation included in the fluorescence image FL.

In the fluorescence image display mode FM in which the fluorescence image FP is displayed in the composite image CP, the image processing circuit 30 outputs the composite image CP resulting from synthesizing the fluorescence image FP and narrow-band images NAP and NBP.

In the fluorescence image non-display mode NFM, the image processing circuit 30 outputs the composite image CP resulting from synthesizing only the narrow-band images NAP and NBP.

In the fluorescence image non-display mode NFM in which the fluorescence image FP is not displayed, the red (R) channel component is missing, and therefore it may be possible to assign the narrow-band image NAP and NBP signals or another predetermined signal to the red (R) channel, multiply the narrow-band image NAP and NBP signals by predetermined weighting factors respectively and synthesize the signals so that the composite image CP as a whole is not tinged blue.

Furthermore, in the fluorescence image non-display mode NFM, instead of totally hiding the fluorescence image FP, the gain of the fluorescence image FP may be reduced by a predetermined amount. In other words, the endoscope apparatus 1 may also include a fluorescence image weak display mode in which the signal level of the fluorescence image FP is lowered and the fluorescence image FP is synthesized with another image so that the composite image is outputted.

That is, the image processing circuit 30 generates a first image signal by the auto fluorescence FL emitted from the connective tissue through the excitation light EX, a second image signal by the first narrow-band light NBa, and a third image signal by the second narrow-band light NBb, and when there is a predetermined instruction such as an instruction of the fluorescence image weak display mode, the image processing circuit 30 outputs a fluorescence image FP with the gain of each signal reduced by a predetermined amount and the narrow-band images NAP and NBP without the gain of each signal being reduced by a predetermined amount.

As described above, the configuration shown in the present modification example can also obtain operations and effects similar to those of the aforementioned embodiment and realize an observation without being distorted considerably by halation or the like.

### (Modification example 4)

In the aforementioned embodiment and modification examples, the operator switches between the normal-light observation mode and the ESD mode, but the light source device may time-sequentially and consecutively emit the white color light WL, excitation light EX, two narrow-band light beams NBa and NBb.

For example, by sequentially driving the white color LED 16a, the excitation light LED 16b, the first narrow-band light LED 16c and the second narrow-band light LED 16d in the LED illumination section 12 in Fig. 1, and consecutively receiving reflected light of the white color light WL, the auto fluorescence FL, the reflected light of the first narrow-band light NBa and the reflected light of the second narrow-band light NBb in that order, the image processing circuit 30 generates the respective images.

The image processing circuit 30 then generates a first image for normal-light observation based on the reflected light of the white color light WL, and generates the composite image CP generated from the auto fluorescence FL, the reflected light of the first narrow-band light NBa, and the reflected light of the second narrow-band light NBb as a second image. The image processing circuit 30 arranges the first image and the second image and simultaneously displays the images on the screen of the observation monitor 5.

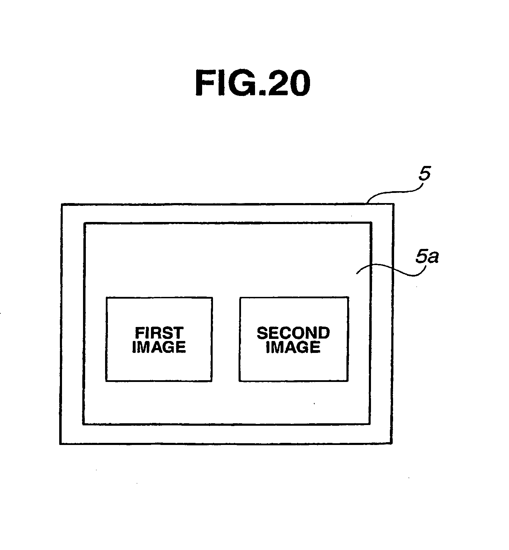

Fig. 20 is a diagram illustrating an example of the display mode of two images simultaneously displayed on the observation monitor 5. As shown in Fig. 20, the first image and the second image are simultaneously displayed side by side on a screen 5a of the observation monitor 5.

That is, the image processing circuit 30 switches between the excitation light EX, the first narrow-band light NBa and the second narrow-band light NBb, and radiates the selected light, generates a first image resulting from synthesizing the three images picked up by the CCD 2, generates a second image from an image obtained by radiating the white color light WL and picked up by the CCD 2 and outputs the image signals of the two images. The first image and the second image are simultaneously displayed on the screen of the observation monitor 5.

According to the present modification example, it is possible to obtain operations and effects similar to those of the aforementioned embodiment, and both of the first image in the normal-light observation mode and the second image in the ESD mode are simultaneously displayed on the screen of the observation monitor 5, and the operator can thereby easily perform ESD.

### (Modification example 5)

In the aforementioned embodiment, white color light is radiated onto a tissue within a body cavity in the normal-light observation mode, whereas in the ESD mode, excitation light EX and two narrow-band light beams NBa and NBb are radiated. Thus, the tinge of an image displayed on the observation monitor 5 considerably differs between the normal-light observation mode and the ESD mode.

Thus, in the present modification example, the difference in tinge of an image displayed on the observation monitor 5 before and after mode switching is reduced.

For this, in the normal-light observation mode, the excitation light EX near a wavelength of 400 nm, the narrow-band light NBb near a wavelength of 600 nm and the white color light WL1 near a wavelength of 400 to 690 nm are sequentially and consecutively emitted.

The CCD 2 which is a color image pickup device picks up images of the sequentially emitted excitation light EX, narrow-band light NBb and white color light WL1, but since light of a wavelength band of 400 to 430 nm is lost in the excitation light cut filter 44, the image processing circuit 30 in the processor 7 converts the contrast to secure color reproducibility of the white color light image.

Furthermore, the image processing circuit 30 assigns the fluorescence image FL for the excitation light EX to the red (R) channel, assigns reflected light of the narrow-band light NBb to the green (G) channel and assigns reflected light of the white color light WL1 to the blue (B) channel to thereby generate composite image CP for normal-light observation.

In this case, in the processor 7, a ratio α between the fluorescence FL and the narrow-band light NBb, that is, a ratio of (intensity of fluorescence FL)/(intensity of narrow-band light NBb) is calculated. The signal of the blue (B) channel of the reflected light of the white color light WL1 is multiplied by the calculated ratio α and the resulting signal is outputted to the blue (B) channel.

That is, the observation monitor 5 displays a composite image CP resulting from synthesizing the image of the blue (B) channel of reflected light of the white color light WU multiplied by the ratio of (intensity of fluorescence FL)/(intensity of narrow-band light NBb), the image of the red (R) channel of the fluorescence image FL and the green (G) channel of the narrow-band light NBb.

The connective tissue of the submucosa 62 emits strong fluorescence FL through the excitation light EX, whereas the submucosa 62 dyed by indigo carmine absorbs the narrow-band light NBb and the reflected light thereof attenuates. Therefore, the ratio α of the fluorescence FL to the narrow-band light NBb considerably changes on the boundary between the mucosal layer 61 and the submucosa 62, and on the boundary between the muscularis propria 63 and the submucosa 62. Thus, by multiplying the blue (B) channel signal of reflected light of the white color light WL1 by the ratio α and synthesizing the three images, in the composite image CP in the normal-light observation mode, the submucosa 62 is displayed more with a tinge of blue than the other tissue within a body cavity.

Therefore, since only the submucosa 62 is drawn in a strong blue color also in the normal-light observation mode, the boundary between the submucosa 62 and the muscularis propria 63 is clearly displayed, allowing the operator to perform treatment while avoiding the risk of perforation in the muscularis propria 63.

Furthermore, when switching between the normal-light observation mode and the ESD mode, tinges of both images do not change considerably, and therefore the operator can perform observation without uncomfortable feeling.

As described above, the configuration shown in the present modification example can also obtain operations and effects similar to those of the aforementioned embodiment and can obtain an observed image without uncomfortable feeling at the time of switching between the normal-light observation mode and the ESD mode.

As described above, according to the aforementioned embodiment and modification examples, it is possible to obtain an endoscope apparatus that displays an image with high visibility of the blood vessel 64 in the depth of the mucous membrane during ESD and also improved visibility of the submucosa 62 on the observation monitor 5.

The ESD is a minimally invasive manipulation that peels off and removes an early-stage cancer or the like before submucosal invasion from the submucosa and thereby removes the lesioned region. According to the aforementioned embodiment and modification examples, the boundary between the submucosa and the muscularis propria is made clear and it is thereby possible to prevent the muscularis propria from being mistakenly removed or perforated. Furthermore, according to the aforementioned embodiment and modification examples, a relatively deep artery can be visualized clearly, preventing the risk of hemorrhage caused by arteriotomy. Thus, the operator can perform ESD safely.

The present invention is not limited to the aforementioned embodiment, but can be modified or altered in various ways without departing from the scope of the present invention.

The present application is filed claiming the priority of Japanese Patent Application No. 2011-087769 filed in Japan on April 11, 2011.

## Claims

1. An endoscope apparatus (1) comprising:
an illumination section (12) adapted to switch light between excitation light (EX) as first narrow-band light (EX), second narrow-band light (NBa) and third narrow-band light (NBb) and irradiate the switched light;
an image pickup section (CCD 2) that is provided in an insertion portion of an endoscope (3), adapted to generate a first image signal (FP) acquired through image pickup by auto fluorescence (FL1, FL2) from a tissue within a body cavity irradiated by the excitation light (EX), generate a second image signal (NAP) acquired through image pickup by return light from the tissue within the body cavity irradiated by the second narrow-band light (NBa), and generate a third image signal (NBP) acquired through image pickup by return light from the tissue within the body cavity irradiated by the third narrow-band light (NBb); and
an image signal generation section (27-30) adapted to assign the first image signal, the second image signal and the third image signal to different colors respectively, and to generate a composite image (CP) by synthesizing the first image signal, the second image signal and the third image signal which are assigned to the different colors;
**characterized in that** the first narrow-band light has a central wavelength of 400 nm that excites a connective tissue in a submucosa (62) of the tissue within the body cavity to emit auto fluorescence (FL1, FL2), the second narrow-band light (NBa) has a central wavelength of 630 nm absorbed by a blood vessel (64) running through the submucosa (62) or a muscularis propria (63) below the submucosa (62) and the third narrow-band light (NBb) has a central wavelength of 600 nm absorbed by a substance (NS) locally injected into the submucosa (62).

2. The endoscope apparatus (1) according to claim 1, further comprising a mode switchover switch (41) adapted to instruct switching between a first mode and a second mode, wherein:
the illumination section (12) can perform illumination with white color light (WL) in addition to the excitation light (EX), the second narrow-band light (NBa), and the third narrow-band light (NBb), and
the illumination section (12) is adapted to switch between the excitation light (EX), the second narrow-band light (NBa), and the third narrow-band light (NBb) and irradiate the switched light when the first mode is instructed with the mode switchover switch (41), and irradiate the white color light (WL) when the second mode is instructed with the mode switchover switch (41).

3. The endoscope apparatus (1) according to claim 1, wherein:
the image signal generation section (27-30) is adapted to assign the second image signal and the third image signal to different colors and generate a composite image (CP) obtained by synthesizing the second image signal and the third image signal which are assigned to the different colors when an instruction not to display the first image signal is received, and assign the first image signal, the second image signal and the third image signal to different colors and generate a composite image (CP) obtained by synthesizing the first image signal, the second image signal and the third image signal which are assigned to the different colors when an instruction not to display the first image signal is not received.

4. The endoscope apparatus (1) according to claim 3, wherein the instruction for not displaying the first image signal is executed based on an operation state of a predetermined switch, an output state of a treatment instrument (SI), or a state of halation included in an image based on the first image signal.

5. The endoscope apparatus (1) according to claim 4, wherein the image signal generation section (27-30) comprises a halation detection section (30a) adapted to detect a halation state included in an image based on the first image signal, and
when the halation detection section (30a) detects the halation, an instruction for not displaying the first image signal is executed.

6. The endoscope apparatus (1) according to claim 1, wherein:
the image signal generation section (27-30) is adapted to lower a gain of the first image signal by a predetermined amount based on predetermined instruction information.

7. The endoscope apparatus (1) according to claim 6, wherein the predetermined instruction information is provided based on an operation state of a predetermined switch, an output state of a treatment instrument (SI) or a halation state included in an image based on the first image signal.

8. The endoscope apparatus (1) according to claim 1, wherein the second narrow-band light (NBa) is light of a wavelength band on a longer wavelength side than the third narrow-band light (NBb) and less susceptible to influences of scattering or absorption on a surface of the tissue within the body cavity.

9. The endoscope apparatus (1) according to claim 1, wherein:
the illumination section (12) is adapted to perform illumination with white color light (WL) in addition to the excitation light (EX), the second narrow-band light (NBa), and the third narrow-band light (NBb), and
the image signal generation section (27-30) is adapted to generate a white color light image acquired through image pickup by the image pickup section (CCD 2) by irradiation of the white color light (WL), and output the composite image (CP) and the white color light image such that the composite image (CP) and the white color light image are simultaneously displayed on a screen (5a) of a display apparatus (5).

10. The endoscope apparatus (1) according to claim 1, wherein:
the substance (NS) is indigo carmine, and
the third narrow-band light (NBb) is narrow-band light corresponding to an absorption peak of a light absorption characteristic of the indigo carmine.

11. The endoscope apparatus (1) according to claim 1, wherein the image pickup section (CCD 2) comprises an excitation light cut filter (44) for intercepting the excitation light (EX).

12. The endoscope apparatus (1) according to claim 1, further comprising a display section (5) adapted to display an image resulting from synthesizing the plurality of images generated by the image signal generation section (27-30).

13. The endoscope apparatus (1) according to claim 1, wherein the illumination section (12) comprises a band limiting section (104) adapted to allow light from a light source (4) to pass therethrough and emit the excitation light (EX) near a wavelength of 400 nm, the second narrow-band light (NBa) near a wavelength of 630 nm, and the third narrow-band light (NBb) near a wavelength of 600 nm as frame sequential light.

14. The endoscope apparatus (1) according to claim 1, wherein the illumination section (12) comprises a light-emitting element (16a-16d) adapted to emit the excitation light (EX) near a wavelength of 400 nm, the second narrow-band light (NBa) near a wavelength of 630 nm, and the third narrow-band light (NBb) near a wavelength of 600 nm as frame sequential light.

15. The endoscope apparatus (1) according to claim 1, wherein
the illumination section (12) is adapted to perform illumination with white color light (WL) in addition to the excitation light (EX), the second narrow-band light (NBa), and the third narrow-band light (NBb), and
the image signal generation section (27-30) is adapted to assign a white color light image signal acquired by the image pickup section (CCD 2) through image pickup of the tissue within the body cavity irradiated by the white color light (WL), the first image signal and the third image signal to different colors respectively, and generate a composite image (CP) by synthesizing the white color light image signal, the first image signal and the third image signal which are assigned to the different colors.

16. The endoscope apparatus (1) according to claim 15, further comprising
an intensity ratio calculation section adapted to calculate an intensity ratio of the auto fluorescence (FL1, FL2) to the return light from the tissue within the body cavity irradiated by the third narrow-band light (NBb),
wherein the image signal generation section (27-30) is adapted to assign the first image signal, the third image signal and a white color light image signal subjected to multiplication by the intensity ratio to different colors respectively, and generate a composite image (CP) by synthesizing the white color light image signal, the first image signal and the third image signal which are assigned to the different colors.

17. The endoscope apparatus (1) according to claim 16, wherein
the image signal generation section (27-30) is adapted to assign the white color light image signal to a blue color, the first image signal to a red color and the third image signal to a green color.

## Patentansprüche

1. Endoskopgerät (1), das umfasst:
einen Beleuchtungsabschnitt (12), der dazu eingerichtet ist, Licht zwischen Anregungslicht (EX) als erstes Schmalbandlicht (EX), zweites Schmalbandlicht (NBa) und drittes Schmalbandlicht (NBb) umzuschalten und das umgeschaltete Licht abzustrahlen;
einen Bildaufnahmeabschnitt (CCD2), der in einem Einführabschnitt eines Endoskops (3) vorgesehen ist, dazu eingerichtet ist, ein erstes Bildsignal (FP) zu erzeugen, das durch die Bildaufnahme durch Autofluoreszenz (FL1, FL2) von einem durch das Anregungslicht (EX) bestrahlten Gewebe in einer Körperhöhlung aufgenommen wird, ein zweites Bildsignal (NAP) zu erzeugen, das durch die Bildaufnahme durch Licht, das von dem durch das zweite Schmalbandlicht (NBa) bestrahlte Gewebe in der Körperhöhlung zurückgestreut wird, aufgenommen wird, und ein drittes Bildsignal (NBP) zu erzeugen, das durch die Bildaufnahme durch Licht, das von dem durch das dritte Schmalbandlicht (NBb) bestrahlte Gewebe in der Körperhöhlung zurückgestreut wird, aufgenommen wird; und
einen Bildsignalerzeugungsabschnitt (27 - 30), der dazu eingerichtet ist, das erste Bildsignal, das zweite Bildsignal und das dritte Bildsignal jeweils verschiedenen Farben zuzuordnen und ein zusammengesetztes Bild (CP) zu erzeugen, indem das erste Bildsignal, das zweite Bildsignal und das dritte Bildsignal, die den verschiedenen Farben zugeordnet sind, synthetisiert werden;
**dadurch gekennzeichnet, dass** das erste Schmalbandlicht eine zentrale Wellenlänge von 400 nm hat, die ein verbundenes Gewebe in einer Submucosa (62) des Gewebes innerhalb der Körperhöhlung anregt, um Autofluoreszenz (FL1, FL2) zu emittieren, das zweite Schmalbandlicht (NBa) eine zentrale Wellenlänge von 630 nm hat, die durch ein Blutgefäß (64), das durch die Submucosa (62) oder eine Muscularis propria (63) unter der Submucosa (62) verläuft, absorbiert wird, und das dritte Schmalbandlicht (NBb) eine zentrale Wellenlänge von 600 nm hat, die durch eine Substanz (NS) absorbiert wird, die lokal in die Submucosa (62) injiziert wird.

2. Endoskopgerät (1) gemäß Anspruch 1, das ferner einen Moduswechselschalter (41) umfasst, der dazu eingerichtet ist, eine Schaltung zwischen einem ersten Modus und einem zweiten Modus zu instruieren, wobei:
der Beleuchtungsabschnitt (12) zusätzlich zu einer Beleuchtung mit dem Anregungslicht (EX), dem zweiten Schmalbandlicht (NBa) und dem dritten Schmalbandlicht (NBb) eine Beleuchtung mit Licht weißer Farbe (WL) durchführen kann, und
der Beleuchtungsabschnitt (12) dazu eingerichtet ist, zwischen dem Anregungslicht (EX), dem zweiten Schmalbandlicht (NBa) und dem dritten Schmalbandlicht (NBb) umzuschalten und das umgeschaltete Licht abzustrahlen, wenn der erste Modus mit dem Moduswechselschalter (41) instruiert wird, und das Licht weißer Farbe (WL) abzustrahlen, wenn der zweite Modus mit dem Moduswechselschalter (41) instruiert wird.

3. Endoskopgerät (1) gemäß Anspruch 1, wobei:
der Bildsignalerzeugungsabschnitt (27 - 30) dazu eingerichtet ist, das zweite Bildsignal und das dritte Bildsignal verschiedenen Farben zuzuordnen und ein zusammengesetztes Bild (CP) zu erzeugen, das durch Synthetisieren des zweiten Bildsignals und des dritten Bildsignals, die den verschiedenen Farben zugeordnet sind, erhalten wird, wenn eine Anweisung, das erste Bildsignal nicht anzuzeigen, empfangen wird, und das erste Bildsignal, das zweite Bildsignal und das dritte Bildsignal verschiedenen Farben zuzuordnen und ein zusammengesetztes Bild (CP) zu erzeugen, das durch Synthetisieren des ersten Bildsignals, des zweiten Bildsignals und des dritten Bildsignals, die den verschiedenen Farben zugeordnet sind, erhalten wird, wenn eine Anweisung, das erste Bildsignal nicht anzuzeigen, nicht empfangen wird.

4. Endoskopgerät (1) gemäß Anspruch 3, wobei die Anweisung zum Nicht-Anzeigen des ersten Bildsignals basierend auf einem Betriebszustand eines vorbestimmten Schalters, einem Ausgabezustand eines Behandlungsinstruments (SI) oder einem Zustand eines Lichthofs ausgeführt wird, der in einem auf dem ersten Bildsignal basierenden Bild enthalten ist.

5. Endoskopgerät (1) gemäß Anspruch 4, wobei der Bildsignalerzeugungsabschnitt (27 - 30) einen Lichthoferfassungsabschnitt (30a) umfasst, der dazu eingerichtet ist, einen Lichthofzustand zu erfassen, der in einem auf dem ersten Bildsignal basierenden Bild enthalten ist, und
wenn der Lichthoferfassungsabschnitt (30a) den Lichthof erfasst, eine Anweisung zum Nicht-Anzeigen des ersten Bildsignals ausgeführt wird.

6. Endoskopgerät (1) gemäß Anspruch 1, wobei:
der Bildsignalerzeugungsabschnitt (27 - 30) dazu eingerichtet ist, basierend auf einer vorbestimmten Anweisungsinformation eine Verstärkung des ersten Bildsignals um einen vorbestimmten Betrag zu verringern.

7. Endoskopgerät (1) gemäß Anspruch 6, wobei die vorbestimmte Anweisungsinformation basierend auf einem Betriebszustand eines vorbestimmten Schalters, einem Ausgabezustand eines Behandlungsinstruments (SI) oder einem Zustand eines Lichthofs bereitgestellt wird, der in einem auf dem ersten Bildsignal basierenden Bild enthalten ist.

8. Endoskopgerät (1) gemäß Anspruch 1, wobei das zweite Schmalbandlicht (NBa) Licht eines Wellenlängenbands ist, das auf einer längeren Wellenlängenseite liegt als das dritte Schmalbandlicht (NBb) und weniger anfällig für Streuungs- oder Absorptionseinflüsse an einer Oberfläche des Gewebes innerhalb der Körperhöhlung ist.

9. Endoskopgerät (1) gemäß Anspruch 1, wobei:
der Beleuchtungsabschnitt (12) dazu eingerichtet ist, zusätzlich zu einer Beleuchtung mit dem Anregungslicht (EX), dem zweiten Schmalbandlicht (NBa) und dem dritten Schmalbandlicht (NBb) eine Beleuchtung mit Licht weißer Farbe (WL) durchzuführen, und
der Bildsignalerzeugungsabschnitt (27 - 30) dazu eingerichtet ist, ein Weißlichtbild zu erzeugen, das durch Bildaufnahme durch den Bildaufnahmeabschnitt (CCD2) durch Bestrahlung mit dem Licht weißer Farbe (WL) aufgenommen wird, und das zusammengesetzte Bild (CP) und das Weißlichtbild auszugeben, so dass das zusammengesetzte Bild (CP) und das Weißlichtbild gleichzeitig auf einem Bildschirm (5a) eines Anzeigegeräts (5) angezeigt werden.

10. Endoskopgerät (1) gemäß Anspruch 1, wobei:
die Substanz (NS) Indigokarmin ist, und
das dritte Schmalbandlicht (NBb) ein Schmalbandlicht ist, das einem Absorptionsmaximum einer Lichtabsorptionslinie von Indigokarmin entspricht.

11. Endoskopgerät (1) gemäß Anspruch 1, wobei der Bildaufnahmeabschnitt (CCD 2) einen Anregungslichtherausschneidefilter (44) zum Auffangen des Anregungslichts (EX) umfasst.

12. Endoskopgerät (1) gemäß Anspruch 1, das ferner einen Anzeigeabschnitt (5a) umfasst, der dazu eingerichtet ist, ein Bild anzuzeigen, das aus einer Synthetisierung der Mehrzahl von Bildern resultiert, die durch den Bildsignalerzeugungsabschnitt (27 - 30) erzeugt werden.

13. Endoskopgerät (1) gemäß Anspruch 1, wobei der Beleuchtungsabschnitt (12) einen Bandbegrenzungsabschnitt (104) umfasst, der dazu eingerichtet ist, Licht von einer Lichtquelle (4) passieren zu lassen und das Anregungslicht (EX) in der Nähe einer Wellenlänge von 400 nm, das zweite Schmalbandlicht (NBa) in der Nähe einer Wellenlänge von 630 nm und das dritte Schmalbandlicht (NBb) in der Nähe einer Wellenlänge von 600 nm als Bildfolgelicht zu emittieren.

14. Endoskopgerät (1) gemäß Anspruch 1, wobei der Beleuchtungsabschnitt (12) ein Licht emittierendes Element (16a -16d) umfasst, das dazu eingerichtet ist, das Anregungslicht (EX) in der Nähe einer Wellenlänge von 400 nm, das zweite Schmalbandlicht (NBa) in der Nähe einer Wellenlänge von 630 nm und das dritte Schmalbandlicht (NBb) in der Nähe einer Wellenlänge von 600 nm als Bildfolgelicht zu emittieren.

15. Endoskopgerät (1) gemäß Anspruch 1, wobei
der Beleuchtungsabschnitt (12) dazu eingerichtet ist, zusätzlich zu einer Beleuchtung mit dem Anregungslicht (EX), dem zweiten Schmalbandlicht (NBa) und dem dritten Schmalbandlicht (NBb) eine Beleuchtung mit Licht weißer Farbe (WL) durchzuführen, und
der Bildsignalerzeugungsabschnitt (27 - 30) dazu eingerichtet ist, ein Weißlichtbildsignal, das durch den Bildaufnahmeabschnitt (CCD2) durch die Aufnahme eines Bilds von dem mit dem Licht weißer Farbe (WL) bestrahlten Gewebe innerhalb der Körperhöhlung aufgenommen wird, das erste Bildsignal und das dritte Bildsignal jeweils verschiedenen Farben zuzuordnen und durch Synthetisieren des Weißlichtbildsignals, des ersten Bildsignals und des dritten Bildsignals, die den verschiedenen Farben zugeordnet sind, ein zusammengesetztes Bild (CP) zu erzeugen.

16. Endoskopgerät (1) gemäß Anspruch 15, das ferner umfasst
einen Intensitätsverhältnisberechnungsabschnitt, der dazu eingerichtet ist, ein Intensitätsverhältnis zwischen der Autofluoreszenz (FL1, FL2) und dem zurückgestreuten Lichts von dem mit dem dritten Schmalbandlicht (NBb) bestrahlten Gewebe innerhalb der Körperhöhlung zu berechnen,
wobei der Bildsignalerzeugungsabschnitt (27 - 30) dazu eingerichtet ist, das erste Bildsignal, das dritte Bildsignal und ein Weißlichtbildsignal, das einer Multiplikation mit dem Intensitätsverhältnis unterzogen wurde, jeweils verschiedenen Farben zuzuordnen, und durch Synthetisieren des Weißlichtbildsignals, des ersten Bildsignals und des dritten Bildsignals, die den verschiedenen Farben zugeordnet sind, ein zusammengesetztes Bild (CP) zu erzeugen.

17. Endoskopgerät (1) gemäß Anspruch 16, wobei
der Bildsignalerzeugungsabschnitt (27 - 30) dazu eingerichtet ist, das Weißlichtbildsignal einer blauen Farbe, das erste Bildsignal einer roten Farbe und das dritte Bildsignal einer grünen Farbe zuzuordnen.

## Revendications

1. Appareil (1) d'endoscope comprenant:
une section d'éclairage (12) adaptée pour commuter une lumière entre une lumière d'excitation (EX) en tant que première lumière à bande étroite (EX), deuxième lumière à bande étroite (NBa) et troisième lumière à bande étroite (NBb) et irradier la lumière commutée;
une section (CCD 2) de capture d'image qui est prévue dans une partie d'insertion d'un endoscope (3), adaptée pour générer un premier signal d'image (FP) acquis par l'intermédiaire d'une capture d'image par auto fluorescence (FL1, FL2) à partir d'un tissu à l'intérieur d'une cavité de corps irradiée par la lumière d'excitation (EX), générer un deuxième signal d'image (NAP) acquis par l'intermédiaire d'une capture d'image par la lumière de retour depuis le tissu à l'intérieur de la cavité de corps irradiée par la deuxième lumière à bande étroite (NBa), et générer un troisième signal d'image (NBP) acquis par l'intermédiaire d'une capture d'image par la lumière de retour depuis le tissu à l'intérieur de la cavité de corps irradiée par la troisième lumière à bande étroite (NBb); et
une section (27 à 30) de génération de signal d'image adaptée pour attribuer le premier signal d'image, le deuxième signal d'image et le troisième signal d'image à différentes couleurs, respectivement, et pour générer une image composite (CP) en synthétisant le premier signal d'image, le deuxième signal d'image et le troisième signal d'image qui sont attribués aux différentes couleurs;
**caractérisé en ce que** la première lumière à bande étroite possède une longueur d'onde centrale de 400 nm qui excite un tissu conjonctif dans une sous-muqueuse (62) du tissu à l'intérieur de la cavité de corps pour émettre une auto fluorescence (FL1, FL2), la deuxième lumière à bande étroite (NBa) possède une longueur d'onde centrale de 630 nm absorbée par un vaisseau sanguin (64) courant à travers la sous-muqueuse (62) ou une couche longitudinale (63) au-dessous de la sous-muqueuse (62), et la troisième lumière à bande étroite (NBb) possède une longueur d'onde centrale de 600 nm absorbée par une substance (NS) injectée localement dans la sous-muqueuse (62).

2. Appareil (1) d'endoscope selon la revendication 1, comprenant en outre un commutateur-inverseur de mode (41) adapté pour instruire une commutation entre un premier mode et un deuxième mode, dans lequel:
la section d'éclairage (12) peut réaliser un éclairage avec une lumière de couleur blanche (WL) en plus de la lumière d'excitation (EX), de la deuxième lumière à bande étroite (NBa), et de la troisième lumière à bande étroite (NBb), et
la section d'éclairage (12) est adaptée pour commuter entre la lumière d'excitation (EX), la deuxième lumière à bande étroite (NBa), et la troisième lumière à bande étroite (NBb) et irradier la lumière commutée lorsque le premier mode est instruit avec le commutateur-inverseur de mode (41), et irradier la lumière de couleur blanche (WL) lorsque le deuxième mode est instruit avec le commutateur-inverseur de mode (41).

3. Appareil (1) d'endoscope selon la revendication 1, dans lequel:
la section (27 à 30) de génération de signal d'image est adaptée pour attribuer le deuxième signal d'image et le troisième signal d'image à différentes couleurs et générer une image composite (CP) obtenue en synthétisant le deuxième signal d'image et le troisième signal d'image qui sont attribués aux différentes couleurs lorsqu'une instruction de ne pas afficher le premier signal d'image est reçue, et attribuer le premier signal d'image, le deuxième signal d'image et le troisième signal d'image à différentes couleurs et générer une image composite (CP) obtenue en synthétisant le premier signal d'image, le deuxième signal d'image et le troisième signal d'image qui sont attribués aux différentes couleurs lorsqu'une instruction de ne pas afficher le premier signal d'image n'est pas reçue.

4. Appareil (1) d'endoscope selon la revendication 3, dans lequel l'instruction de ne pas afficher le premier signal d'image est exécutée sur la base d'un état opérationnel d'un commutateur prédéterminé, d'un état de sortie d'un instrument de traitement (SI), ou d'un état de halo contenu dans une image sur la base du premier signal d'image.

5. Appareil (1) d'endoscope selon la revendication 4, dans lequel la section (27 à 30) de génération de signal d'image comprend une section (30a) de détection de halo adaptée pour détecter un état de halo contenu dans une image sur la base du premier signal d'image, et
lorsque la section (30a) de détection de halo détecte le halo, une instruction de ne pas afficher le premier signal d'image est exécutée.

6. Appareil (1) d'endoscope selon la revendication 1, dans lequel :
la section (27 à 30) de génération de signal image est adaptée pour abaisser un gain du premier signal d'image d'une quantité prédéterminée sur la base d'une information d'instruction prédéterminée.

7. Appareil (1) d'endoscope selon la revendication 6, dans lequel l'information d'instruction prédéterminée est prévue sur la base d'un état opérationnel d'un commutateur prédéterminé, d'un état de sortie d'un instrument de traitement (SI) ou d'un état de halo contenu dans une image sur la base du premier signal d'image.

8. Appareil (1) d'endoscope selon la revendication 1, dans lequel la deuxième lumière à bande étroite (NBa) est une lumière d'une bande de longueur d'onde sur un côté de longueur d'onde plus longue que la troisième lumière à bande étroite (NBb) et moins sensible aux influences de diffusion ou d'absorption sur une surface du tissu à l'intérieur de la cavité de corps.

9. Appareil (1) d'endoscope selon la revendication 1, dans lequel:
la section d'éclairage (12) est adaptée pour réaliser un éclairage avec une lumière de couleur blanche (WL) en plus de la lumière d'excitation (EX), de la deuxième lumière à bande étroite (NBa) et de la troisième lumière à bande étroite (NBb), et
la section (27 à 30) de génération de signal d'image est adaptée pour générer une image à la lumière de couleur blanche acquise par l'intermédiaire d'une capture d'image par la section (CCD 2) de capture d'image par irradiation de la lumière de couleur blanche (WL), et délivrer en sortie l'image composite (CP) et l'image à la lumière de couleur blanche d'une manière telle que l'image composite (CP) et l'image à la lumière de couleur blanche sont simultanément affichées sur un écran (5a) d'un appareil d'affichage (5).

10. Appareil (1) d'endoscope selon la revendication 1, dans lequel :
la substance (NS) est du carmin d'indigo, et
la troisième lumière à bande étroite (NBb) est une lumière à bande étroite correspondant à une crête d'absorption d'une caractéristique d'absorption de lumière du carmin d'indigo.

11. Appareil (1) d'endoscope selon la revendication 1, dans lequel la section (CCD 2) de capture d'image comprend un filtre (44) de coupure de lumière d'excitation destiné à intercepter la lumière d'excitation (EX).

12. Appareil (1) d'endoscope selon la revendication 1, comprenant en outre une section d'affichage (5) adaptée pour afficher une image résultant de la synthétisation de la pluralité d'images générées par la section (27 à 30) de génération de signal d'image.

13. Appareil (1) d'endoscope selon la revendication 1, dans lequel la section d'éclairage (12) comprend une section (104) de limitation de bande adaptée pour permettre à la lumière provenant d'une source de lumière (4) de passer à travers et d'émettre la lumière d'excitation (EX) proche d'une longueur d'onde de 400 nm, la deuxième lumière à bande étroite (NBa) proche d'une longueur d'onde de 630 nm, et la troisième lumière à bande étroite (NBb) proche d'une longueur d'onde de 600 nm en tant que lumière à séquence de trames.

14. Appareil (1) d'endoscope selon la revendication 1, dans lequel la section d'éclairage (12) comprend un élément d'émission de lumière (16a à 16d) adapté pour émettre la lumière d'excitation (EX) proche d'une longueur d'onde de 400 nm, la deuxième lumière à bande étroite (NBa) proche d'une longueur d'onde de 630 nm, et la troisième lumière à bande étroite (NBb) proche d'une longueur d'onde de 600 nm en tant que lumière à séquence de trames.

15. Appareil (1) d'endoscope selon la revendication 1, dans lequel
la section d'éclairage (12) est adaptée pour réaliser un éclairage avec une lumière de couleur blanche (WL) en plus de la lumière d'excitation (EX), de la deuxième lumière à bande étroite (NBa), et de la troisième lumière à bande étroite (NBb), et
la section (27 à 30) de génération de signal image est adaptée pour attribuer un signal d'image de lumière de couleur blanche acquis par la section (CCD 2) de capture d'image par l'intermédiaire d'une capture d'image du tissu à l'intérieur de la cavité de corps irradiée par la lumière de couleur blanche (WL), le premier signal d'image et le troisième signal d'image à des couleurs différentes, respectivement, et générer une image composite (CP) en synthétisant le signal d'image de lumière de couleur blanche, le premier signal d'image et le troisième signal d'image qui sont attribués aux différentes couleurs.

16. Appareil (1) d'endoscope selon la revendication 15, comprenant en outre
une section de calcul de rapport d'intensité adaptée pour calculer un rapport d'intensité de l'auto fluorescence (FL1, FL2) par rapport à la lumière de retour depuis le tissu à l'intérieur de la cavité de corps irradiée par la troisième lumière à bande étroite (NBb),
dans lequel la section (27 à 30) de génération de signal d'image est adaptée pour attribuer le premier signal d'image, le troisième signal d'image et un signal d'image de lumière de couleur blanche soumis à la multiplication par le rapport d'intensité aux différentes couleurs, respectivement, et générer une image composite (CP) en synthétisant le signal d'image de lumière de couleur blanche, le premier signal d'image et le troisième signal d'image qui sont attribués aux différentes couleurs.

17. Appareil (1) d'endoscope selon la revendication 16, dans lequel
la section (27 à 30) de génération de signal d'image est adaptée pour attribuer le signal d'image de lumière de couleur blanche à une couleur bleue, le premier signal d'image à une couleur rouge et le troisième signal d'image à une couleur verte.
